# EUROPEAN PATENT APPLICATION

(11) **EP 1 749 831 A1**
(43) Date of publication of application: **07.02.2007**
(21) Application number: 05741689.3
(22) Date of filing: 08.04.2005
(51) Int. Cl.: C07F 15/00, A61K 31/282, A61P 35/00

(54) **DEMETHYL-CANTHARIDIN PLATINUM COMPLEX ISOMERS AND THEIR USE**

(30) Priority: 08.04.2004 CN 200410033701
(71) Applicant: THE CHINESE UNIVERSITY OF HONG KONG, Hong Kong SAR (CN)
(72) Inventor: HO, Yee-Ping, Fo Tan, N.T. Hong Kong (CN); AU-YEUNG, Steve, C., F., Hong Kong (CN); TO, Kin Wah, Kowloon, Hong Kong (CN); WANG, Xinning, Funing County, Jiangsu 224400 (CN)
(74) Representative: Krauss, Jan
(86) International application number: PCT/CN2005/000463
(87) International publication number: WO 2005/097813

(57) **Abstract**

The present invention provides a stereo-isomer of the demethylcantharidin platinum complex of formula I and use thereof. The stereo-isomer inhibits the growth of cisplatin, carboplatin or oxaliplatin-sensitive and -resistant tumorous cells. The invention also describes a pharmaceutical composition comprising the stereo-isomer of the demethylcantharidin platinum complex and use thereof.

## Description

### TECHNICAL FIELD OF THE INVENTION

The present invention relates to stereo-isomers of a demethylcantharidin platinum complex and use thereof in inhibiting the growth of tumorous cells.

### BACKGROUND OF THE INVENTION

Demethylcantharidin (DMC) is modified from cantharidin, in which cantharidin is an active ingredient of "blister beetles". Blister beetles have been used as a traditional Chinese medicine (TCM) for the treatment of tumors of human liver, lung, intestinal and digestive tract.

Cisplatin and carboplatin are both platinum-based medicaments clinically used as antitumor drugs worldwide. Common problems associated with these drugs are their toxic side effects and drug resistance.

US6,110,907 granted to the present inventors discloses a DMC platinum complex including the complex of formula I, which has an excellent antitumor activity.

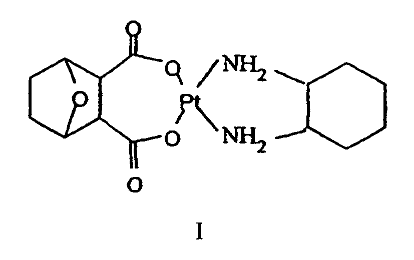

A PCT application PCT/CN01/00885 filed by the present applicant has demonstrated that DMC shows a synergistic effect when used in combination with the DMC platinum complex including the complex of formula I mentioned above or other platinum complexes such as cisplatin, carboplatin and oxaliplatin. However, in the prior art, there has not been any disclosure of the activity of stereo-isomers of the platinum complex of formula 1.

The present invention is provided based on a surprising discovery of the present inventors that some stereo-isomers of the platinum complex of formula I have an excellent activity against tumors.

### SUMMARY OF THE INVENTION

According to an aspect of the present invention, there is provided a stereo-isomer of the demethylcantharidin (DMC) platinum complex of formula I, 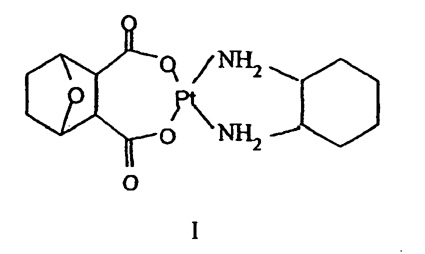 in which the stereo-isomer is a cis-isomer, a trans-S,S(+)-isomer, a trans-R,R(-)-isomer or a trans-racemate.

According to another aspect of the present invention, there is provided use of a stereo-isomer of the DMC platinum complex of formula I in manufacturing a medicament for inhibiting the growth of tumorous cells, in which the stereo-isomer is a cis-isomer, a trans-S,S(+)-isomer, a trans-R,R(-)-isomer or a trans-racemate.

In a preferred embodiment of the invention, the tumorous cells include cisplatin, carboplatin or oxaliplatin-sensitive and -resistant tumorous cells.

In a further preferred embodiment of the invention, the tumorous cells are derived from lung, liver, breast, colon, ovary, cervix, leukemia, lymphoma, or naso-pharyngeal carcinoma.

According to still another aspect of the present invention, there is provided a pharmaceutical composition comprising a stereo-isomer of the demethylcantharidin (DMC) platinum complex of formula I and a pharmaceutically acceptable carrier, in which the stereo-isomer is a cis-isomer, a trans-S,S(+)-isomer, a trans-R,R(-)-isomer or a trans-racemate.

Preferably, the pharmaceutical composition of the invention can be used for the treatment of tumors. The tumors include those sensitive or resistant to cisplatin, carboplatin and oxaliplatin, and preferably include lung, liver, breast, colon, ovary, cervix, leukemia, lymphoma, or naso-pharyngeal carcinoma.

Preferably, the composition of the invention is formulated in a form suitable for topical, oral, intravenous and intramuscular administration.

According to still another aspect of the present invention, there is provided a method of inhibiting the growth of tumorous cells comprising contacting the tumorous cells with a stereo-isomer of the demethylcantharidin platinum complex of formula I, in which the stereo-isomer is a cis-isomer, a trans-S,S(+)-isomer, a trans-R,R(-)-isomer or a trans-racemate. The tumorous cells include cisplatin, carboplatin or oxaliplatin-sensitive and -resistant tumorous cells, and preferably are derived from lung, liver, breast, colon, ovary, cervix, leukemia, lymphoma, or naso-pharyngeal carcinoma.

When used herein, the term "inhibiting (inhibition)" refers to inhibiting (inhibition) *in vitro* and/or *in vivo.*

According to a further aspect of the present invention, there is provided a method for treating a tumor in a subject comprising administering to the subject a therapeutically effective amount of a stereo-isomer of the demethylcantharidin platinum complex of formula I or a pharmaceutical composition containing the same, in which the stereo-isomer is a cis-isomer, a trans-S,S(+)-isomer, a trans-R,R(-)-isomer or a trans-racemate. Preferably, the tumor includes those sensitive or resistant to cisplatin, carboplatin and oxaliplatin, and in particular includes lung, liver, breast, colon, ovary, cervix, leukemia, lymphoma, or naso-pharyngeal carcinoma.

### BRIEF DESCRIPTION OF THE DRAWINGS

Figure 1 shows a circular dichroism spectrum of a stereo-isomer of the DMC platinum complex.

Figure 2 shows a circular dichroism spectrum of the 1,2-DACH stereo-isomer.

Figure 3 shows a circular dichroism spectrum of the S,S-DMC platinum complex (0.25mg/ml), which demonstrates the reproducibility of the spectrum; and

Figure 4 shows a circular dichroism spectrum of DMC (0.1mg/ml), which demonstrates that DMC has no optical activity.

### DETAILED DESCRIPTION OF THE INVENTION

The present invention provides a stereo-isomer of the demethylcantharidin (DMC) platinum complex of formula I, 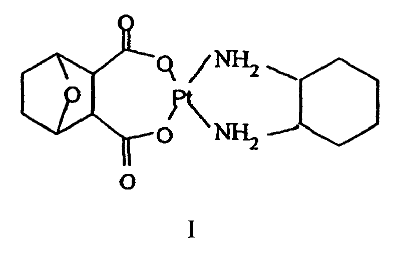 in which the stereo-isomer is a cis-isomer, a trans-S,S(+)-isomer, a trans-R,R(-)-isomer or a trans-racemate.

The present invention also provides use of the stereo-isomer of the DMC platinum complex of formula I in manufacturing medicaments for inhibiting the growth of tumorous cells.

Preferably, the tumorous cells include cisplatin, carboplatin or oxaliplatin-sensitive and -resistant tumorous cells, and are derived from lung, liver, breast, colon, ovary, cervix, leukemia, lymphoma, or naso-pharyngeal carcinoma.

The present invention further provides a pharmaceutical composition comprising a therapeutically effective amount of the stereo-isomer of the demethylcantharidin (DMC) platinum complex of formula I and a pharmaceutically acceptable carrier.

As used herein, a "therapeutically effective amount" is any amount effective to inhibit the tumorous cells or the growth thereof in a subject that typically comprises animals and human.

Methods for determination of a "therapeutically effective amount" are well known to those skilled in the art and depend upon a number of factors including, but not limited to: the type of a subject involved including its gender, age, status and the like, the condition of a disease to be treated and the form of a formulation used. For example, in a pharmaceutical composition, the therapeutically effective amount may be 0.01-99.99%, preferably 0.5-0-99.95%, and more preferably 1-99% of the total amount of the composition, by weight.

The composition of the present invention can be formulated into preparations for therapeutic administration via various routes. For example, the composition may be formulated into forms suitable for topical; oral, intravenous and intramuscular administration. The preparations may be administrated to a subject or patient in need of the treatment via various routes including oral, buccal, rectal, parenteral, Intraabdominal, intradermal, transdermal and intratracheal route, and the like.

Preparations suitable for the topical administration may be in the forms of transdermal dressing, suppository, paste, wash, slurry, gel and the like. A topical preparation may further contain one or more penetrants, thickening agents, diluents, emulsifying agents, dispersants or binding agents. For the transdermal administration, the composition may be formulated or administrated in combination with a penetration enhancer, including chemical and physical penetration enhancers, which can enhance the delivery of compositions through skin. Examples of chemical and physical penetration enhancers are disclosed in, such as, Transdermal Delivery of Drugs, A. F. Kydonieus (ED) 1987 CRL Press; Percutaneous Penetration Enhancers, eds. Smith et al., CRC Press, 1995**;** Lenneruas et al., J Pharm Pharmacol 2002, 54(4): 499-508; Karande et al., Pharm Res 2002, 19(5): 655-60; Vaddi et al., J Pharm Sci 2002 July, 91(7): 1639-51; Ventura et al., J Drug Target 2001; 9(5): 379-93; Shokri et al., Int J Pharm 2001, 228(1-2): 99-107; Suzuki et al., Biol Pharm Bull 2001, 24(6): 698-700; Alberti et al., J Control Release 2001, 71(3): 319- 27; Goldstein et al., Urology 2001; 57(2): 301-5; Kiijavainen et al., Eur J Pharm Sci 2000, 10(2): 97-102; and Tenjarla et al., Int J Pharm 1999, 192(2): 147-58.

Where the composition of the invention is formulated with a compatible chemical penetration enhancer, the enhancer is used in an amount sufficient to enhance the delivery of the complex of the invention through the skin of a subject, such as the delivery of the complex to the system circulation of the subject.

For the oral administration, the complex can be formulated into tablets, powders, granules or capsules alone or in combination with a suitable additive including, but not limited to, conventional additives such as lactose, mannitol, corn starch and potato starch, mistura such as crystalline cellulose, cellulose derivatives, arabic gum, corn starch and gelatin, disintegrants such as corn starch, potato starch and carboxymethycellulose sodium, lubricants such as talc and magnesium stearate and, if desired, diluents, buffers, wetting agents, preservatives and flavouring agents. Preferably, the complex is formulated with a buffer to protect it in the low pH condition within the stomach of a subject. Alternatively, an enteric coating is provided to avoid the precipitation of the complex while passing through the stomach.

The complex of the invention can be formulated into injections by its salvation, suspension or emulsification in a water-soluble or non-water-soluble solution, such as a vegetable oil or like oils, synthetic fatty glyceride, higher fatty acid ester and acryl ethylene glycol. If desired, the complex may be formulated in combination with a conventional additive, such as a solubilizing agent, an isotonizing agent, a suspending agent, an emulsifying agent, a stabilizer and a preservative. Preferred the solubilizing agent includes vitamin E TPGS (d-a-tocopherol polyethylene glycol 1000 succinate), dextrin and the like.

The present invention further provides a method of inhibiting the growth of tumorous cells comprising contacting the tumorous cells with an amount of a stereo-isomer of the demethylcantharidin platinum complex of formula I, in which the stereo-isomer is a cis-isomer, a trans-S,S(+)-isomer, a trans-R,R(-)-isomer or a trans-racemate. Preferably; the tumorous cells include cisplatin, carboplatin or oxaliplatin-sensitive and -resistant tumorous cells, and are derived from lung, liver, breast, colon, ovary, cervix, leukemia, lymphoma, or naso-pharyngeal carcinoma.

When used herein, the term "inhibiting (inhibition)" refers to inhibiting (inhibition) *in vitro* and/or *in vivo.*

The present invention further provides a method for treating tumor in a subject comprising administering to the subject a therapeutically effective amount of the stereo-isomer of the demethylcantharidin platinum complex of formula I or a pharmaceutical composition containing the same. The tumor includes lung, liver, breast, colon, ovary, cervix, leukemia, lymphoma, or naso-pharyngeal carcinoma.

A method for the synthesis of stereo-isomers of the demethylcantharidin platinum complex of the invention is illustrated as follows.

### 1. Preparation of Demethylcantharidin Ligands

Furan and maleic anhydride are dissolved in an organic solvent such as tetrahydrofuran (THF) to obtain an intermediate, followed by addition of Pd-C catalyst. The reaction mixture is then refluxed under aeration of hydrogen to afford the DMC ligand.

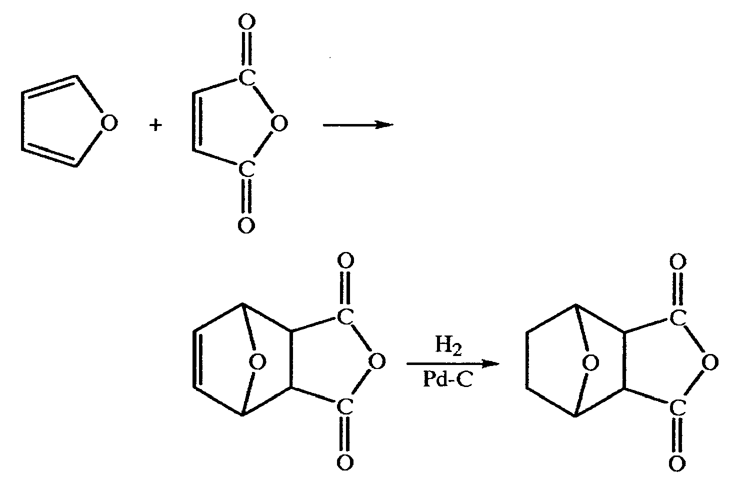

### 2. Preparation of Pt (II) Nitrate derivatives

K₂PtCl₄ is dissolved in water and then reacted with diaminocyclohexane (DACH), followed by addition of silver nitrate to obtain a Pt (II) Nitrate derivative.

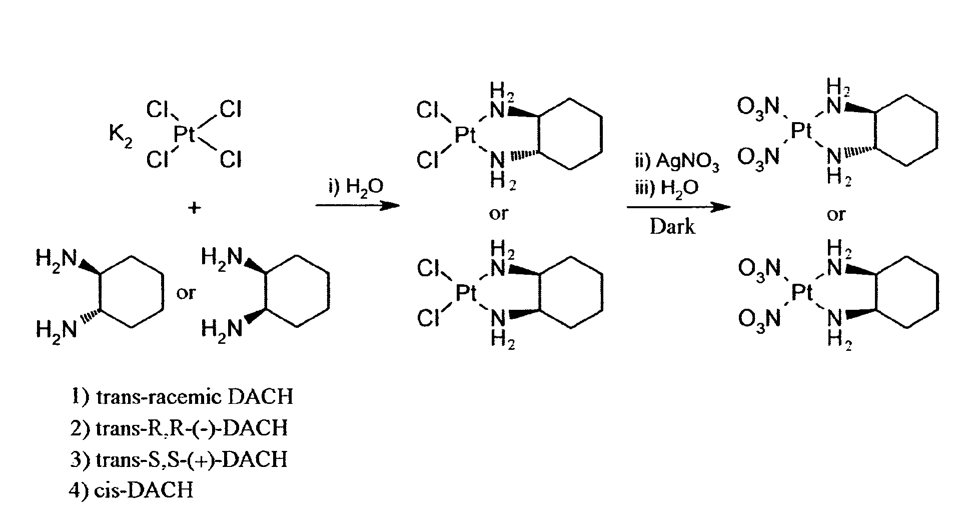

### 3. Synthesis of DMC platinum Complexes

To the Pt (II) Nitrate derivative prepared in step 2 is added the DMC ligand prepared in step 1, followed by NaOH to give the DMC platinum complex.

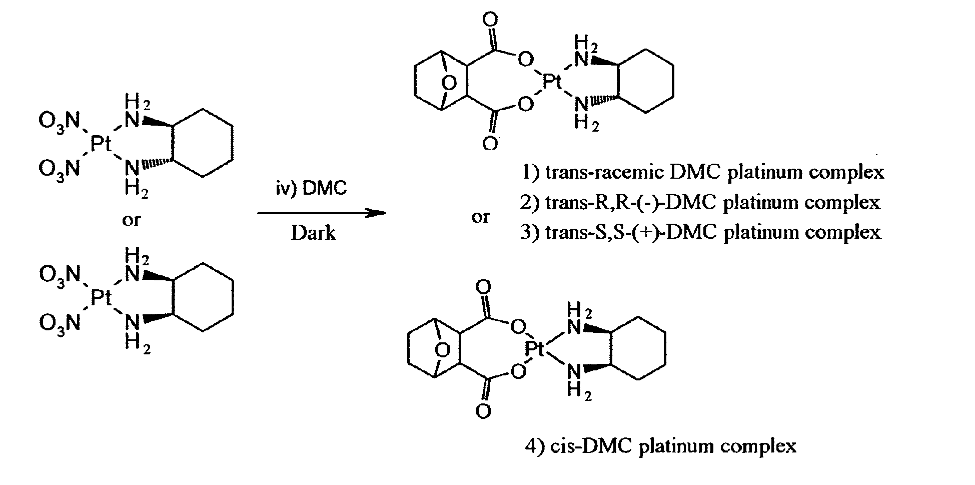

Furan and hydrogen peroxide are commercially available from RDH Company, maleic anhydride and silver nitrate are available from Farco Chemical Supplies, Pd-C (10% activated carbon) is available from BDH Laboratory Supplies, and K₂PtCl₄(II) and various derivatives of diaminocyclohexane are available from Aldrich Chemical Company.

Hereinafter, the present invention will be illustrated in detail with reference to the following examples.

### Example 1

### Preparation of trans-racemic DMC platinum complex

Furan (5.4ml, 0.073 mol) and maleic anhydride (6g, 0.061 mol) were placed into a dry beaker containing THF (8 ml), and stirred at room temperature for 60 minutes. Solvent was removed in vacuo, and the residue was recrystallized in ethyl acetate to afford an intermediate as a white crystal (9.1 g, 88.4% yield). The intermediate (2g, 0.012 mol) was dissolved in ethyl acetate (30 ml) and Pd-C catalyst (10% activated carbon, 0.2g) was then added. The reaction mixture was refluxed with stirring, and hydrogen gas was bubbled into the reaction flask at atmospheric pressure for 16 hours. The Pd-C was then filtered off, and solvent was removed in vacuo to give a DMC ligand (98% yield).
Melting point: 116.9~117.3°C.
IR (KBr, cm⁻¹): 1843, 1784 (C=O), 1237, 1099, 890 (C-O-C).

To a solution of K₂PtCl₄ (1g, 2.410mmol) in water (5ml) was added dropwise a solution of trans-racemic diaminocyclohexane (DACH) (0.2747g, 2.410mmol) in water (1ml) and a yellow precipitate was formed. The mixture was stirred at room temperature for 6~8 hours, filtered, washed successively with water (5ml), ethanol (5ml) and ether (5ml), and then dried in an oven at a temperature of 65°C.

AgNO₃ (0.3579g, 2.1053mmol) and Pt(DACH)Cl₂ (0.4g, 1.0526mmol) were placed into a reaction flask containing water (5ml), stirred in the dark for 18 hours, and then filtered to remove solid AgCl. The DMC ligand as prepared above (0.1768g, 1.0526mmol) and NaOH (0.0842g, 1.1053mmol) were added to the filtrate, and stirred at room temperature for 16 hours to give an offwhite solid. The solid was dried in vacuo, washed successively with ice water (5ml), ethanol (5ml) and ether (5ml), and then dried in an oven at a temperature of 65°C to afford a product of 0.22g (72%).
IR (KBr, cm⁻¹): 3195, 3150 (N-H), 1642, 1608 (C=O), 1396 (COO⁻), 1267, 1214, 1066, 940 (C-O-C).

### Example 2

### Preparation of trans-R,R-(-)-DMC platinum complex

Trans-R,R-(-)-DMC platinum complex was prepared in the same manner as described in Example 1 except that trans-R,R-(-)-DACH was used in place of trans-racemic DACH. Yield was 73%, and the product was decomposed at 200°~250°C.

### Example 3

### Preparation of trans-S,S-(+)-DMC platinum complex

Trans-S,S-(+)-DMC platinum complex was prepared in the same manner as described in Example 1 except that trans-S,S-(+)-DACH was used in place of trans-racemic DACH. Yield was 73%, and the product was decomposed at 200°~250°C.

### Example 4

### Preparation of cis-DMC platinum complex

Cis-DMC platinum complex was prepared in the same manner as described in Example 1 except that cis-DACH is used in place of trans-racemic DACH. Yield was 73%, and the product was decomposed at 200°~250°C.

### Example 5

### Identification of the complexes

### (1) Specific rotatory power of trans-R,R-DMC/-S,S-DMC platinum complex

The specific rotatory power of trans-R,R-DMC/-S,S-DMC platinum complex was determined under the following conditions, and results were as shown in Table 1.
Temperature: 20°C;
Concentration: 0.25mg/ml for trans-R,R-DMC and trans-S,S-DMC platinum complexes; 1mg/ml for trans-R,R-1,2- and trans-S,S-1,2- diaminocyclohexane;
Cell Length: 10cm;
Solvent: water;
Monochromatic Light: the Sodium D-line with a wavelength of 589.3nm.

**Table 1**

| Trial | Trans-R,R-DMC platinum complex [α]_{D} | Trans-S,S-DMC platinum complex [α]_{D} |
|---|---|---|
| 1 | +58.508° | -56.242° |
| 2 | +54.878° | -59.849° |
| 3 | +59.611° | -60.785° |
| 4 | +61.311° | -57.540° |
| 5 | +48.613° | -56.590° |
| 6 | +49.923° | -52.991° |
| 7 | +57.603° | -56.190° |
| 8 | +54.156° | -61.100° |
| **Average** | **+55.575°** | **-57.661°** |

| | | |
|---|---|---|
| [α]_{D} for trans-R,R-1,2-diaminocyclohexane: -32.949°; reference value for Aldrich: -25 [α]_{D} for trans-S,S-1,2- diaminocyclohexane: +34.197°; reference value for Aldrich: +25 | | |

### (2) Circular dichroism spectrum for stereo-isomers of DMC platinum complex, stereo-isomers of 1,2-diaminocyclohexane, and DMC

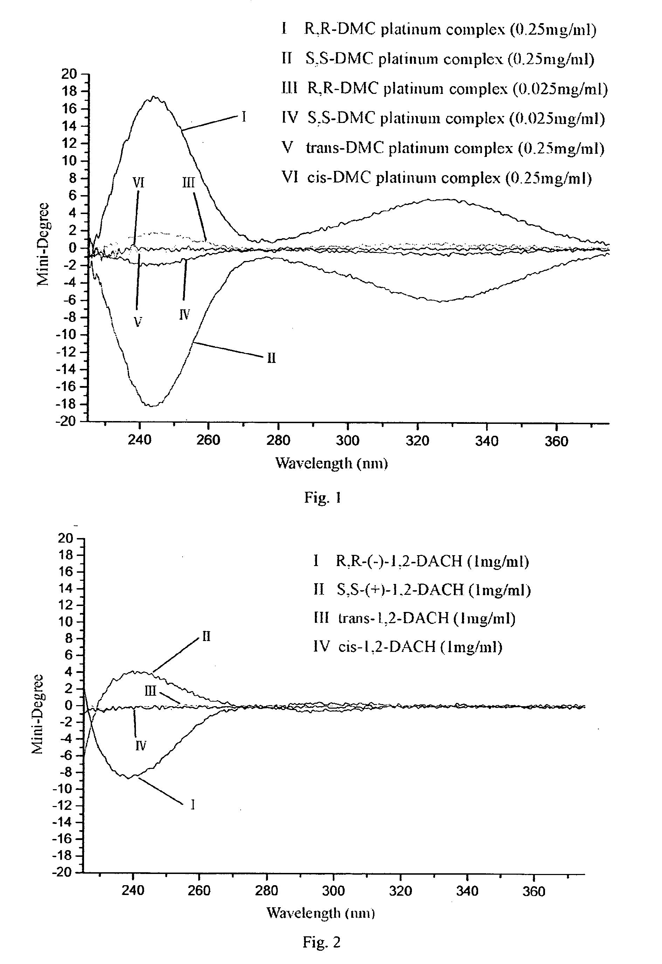

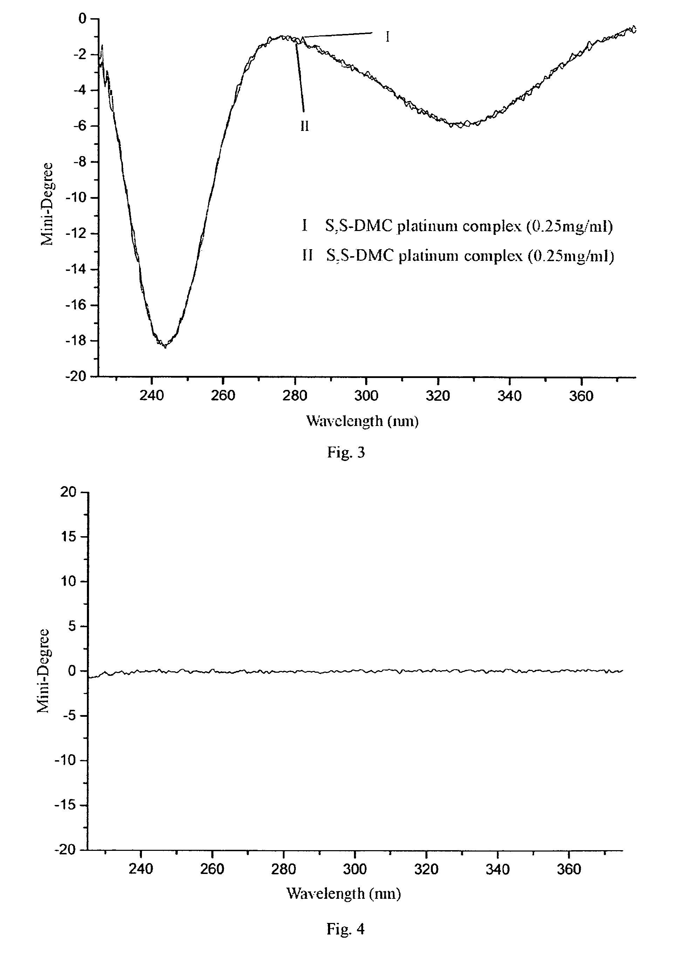

The circular dichroism spectrum for the above compounds was determined under the following conditions, and results were as shown in Figures 1-4.
Instrument: JASCO J-715 Circular Dichroism Spectrometer;
Cell Length: 1.0cm;
Solvent: water;
Temperature: 20°C;
Data mode: CD;
Ch2-mode: HT;
Range: 225-375nm;
Band width: 1.0nm;
Sensitivity: 200mdeg;
Resolution ratio: 0.1nm;
Response: 1sec;
Accumulation: 1;
Speed: 50nm/min.

### (3) Solubility of stereo-isomers of DMC platinum complex

The solubility of stereo-isomers of DMC platinum complex was shown in Table 2.

**Table 2**

| | Trans-isomer | Trans-racemate | Trans-S,S(+)-isomer | Trans-R,R(-)-isomer |
|---|---|---|---|---|
| Solubility (mg/ml) | 5 | 1 | 1 | 1 |

### (4) Conclusion

The following conclusion was reached in view of the results of the specific rotatory power and circular dichroism spectrum mentioned above.
1) As shown in the following Table 3, an interchange in the sign (+/-) of the optical rotation has been observed between the starting material DACH and the product.

**Table 3**

| | R,R-DACH | Trans-R,R(-)-DMC platinum complex | S,S-DACH | Trans-S,S(+)-DMC platinum complex |
|---|---|---|---|---|
| Optical activity | - | + | + | - |

2) The Data for trans-S,S-DACH or trans-R,R-DACH and trans-S,S-DMC platinum complex or trans-R,R-DMC platinum were reproducible.
3) All compounds that have no optical activity, such as DMC, trans-DACH, cis-DACH, trans-DMC platinum complex, cis-DMC platinum complex, were lying on the baseline without showing any signal.

### (5) NMR spectra for stereo-isomers of DMC platinum complex

The NMR spectra of the stereo-isomers of DMC platinum complex as prepared in Examples 1-4 was shown in the following Table 4.
Experimental conditions:
Solvent: D₂O
Instrument: Bruker ARX-500 High Resolution Superconducting FT-NMR Spectrometer (equipped with a 5mm BBI Z-Gradient probehead)
Frequency: ¹H 500.131 MHz
Recording conditions:
1-D Experiments:
   ¹H: D1=1sec, P1=6µsec, DE=88.75µsec
2-D Experiments:
   ¹H: D1=1sec, P1=8.50µsec, DE=237.14µsec
   D0=3µsec, P0=8.50µsec

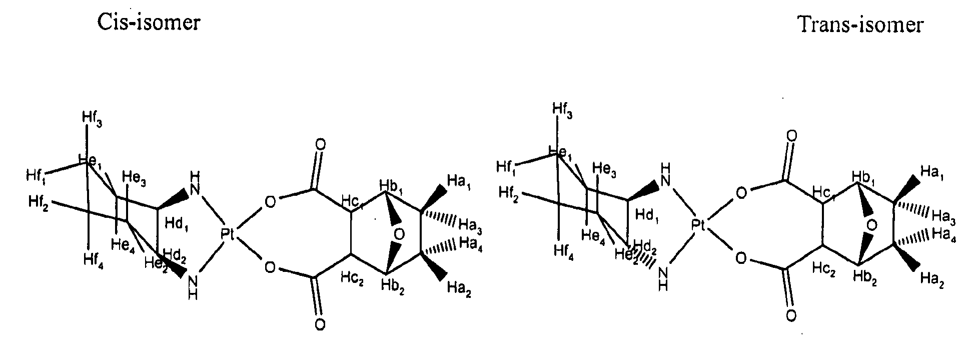

**Table 4**

| DMC platinum complex | | He₁, He₂ | He₃,He₄ | Hf₁, Hf₂ | Hf₃, Hf₄ | Ha₁, Ha₂, Ha₃, Ha₄ | Hb₁, Hb₂ | Hc₁, Hc₂ | Hd₁, Hd₂ |
|---|---|---|---|---|---|---|---|---|---|
| Cis-isomer | δ/ppm J/Hz | 1.82-1.85(m) | 1.59-1.61(m) | 1.82-1.85(m) | 1.39-1.43 (m) | 1.75 (m) | 4.93 (s) | 3.99 (s) | 2.91 (m) |
| Trans-racemate | δ/ppm J/Hz | 2.06 (m) | 1.31 (m) | 1.59 (m) | 1.18 (m) | 1.76 (m) | 4.85 (m) | 3.98 (d) 4.01 (d) 10.8, 10.8 | 2.38 (m) |
| Trans-(R,R)- | δ/ppm J/Hz | 2.05 (m) | 1.31 (m) | 1.59 (m) | 1.17 (m) | 1,76 (m) | 4.85 (m) | 3.97(d) 4.01 (d) 10.9 10.9 | 2.37 (m) |
| Trans-(S,S)- | δ/ppm J/Hz | 2.04 (m) | 1.31 (m) | 1.59 (m) | 1.17 (m) | 1.74 (m) | 4.85 (m) | 3.97(d) 4.01(d) 10.9, 10.9 | 2.37 (m) |

| | | | | | | | | | |
|---|---|---|---|---|---|---|---|---|---|
| s=singlet ; m=multiplet ; d=doublet | | | | | | | | | |

### Example 6

### Test of antitumor activities in vitro

DMC platinum complexes, together with controls cisplatin, carboplatin, and oxaliplatin were screened for an *in vitro* antitumor activity against various tumorous cell lines, using a tetrazolium microculture assay.

Drugs: Drugs to be tested were dissolved in a complete medium at twice the highest concentration to be used, followed by filtration (0.22µm) sterilization. A series of log dilutions were made from each drug solution using complete medium. All drug solutions were prepared 10 minutes before addition to cells.

*In vitro* drug exposure: For each drug to be tested, trypsinized cells were adjusted to a concentration of 2x10⁵ cells/ml in a complete medium, and 0.1ml of the cell suspension was added to each of 24 wells in a 96-well microculture plate. The plate was then incubated at a standard condition for 24 hours in order to allow the cells to be adhered to the bottom of the well and restart exponential growth. To each well of the control culture and drug/concentration combinations, 0.1ml of the complete medium and drug solution were added, respectively. Each drug/concentration combination consisting of four replicates while the control culture consisting of 12 replicates. The twelve replicates of a blank control containing 0.2ml of the complete medium were set aside to account for the background. The cultures were incubated for additional 72 hours. All tests were repeated at least 3 times on different occasions.

MTT assay: The MTT (tetrazolium) assay was used to determine the growth-inhibitory effects of different drugs. A 5mg/ml solution of MTT (3-(4,5-dimethylthiazol-2-yl)-2,5- diphenyltetrazolium bromide) was prepared in phosphate buffer saline (pH 7.4), sterilized by filtration (0.22µm) and stored at -20°C. To each well, 0.02ml of an MTT solution was added, and the plate was incubated for 4 hours under standard conditions. The medium in each well was then removed, and 0.1ml of DMSO (dimethylsulfoxide) was added to each well to solubilize the colored dye produced from the metabolic reduction of MTT. The amount of reduced MTT was measured by spectrophotometric means (570nm).

Data analysis: A mean value of OD was determined for all of the 12 control wells. For each concentration of each drug, A mean OD of the 4 wells was determined. All the mean ODs were subtracted from the blank to account for the background. The "Percent of Control" values were determined and plotted. The concentration of drugs resulting in a 50% growth inhibition (IC50) was determined using the software Prism 3.0 by fitting the graph into sigmoidal dose-response curve. Results were as shown in Table 5.

**Table 5**

| Average growth-inhibitory activity (IC₅₀/µM) of cisplatin, carboplatin, oxaliplatin, DMC, DMC platinum complexes and analogues thereof to tumorous cell lines (n=24 in each independent experiment, and number of experiment performed is indicated in the parenthesis) | | | | | | | | |
|---|---|---|---|---|---|---|---|---|
| Cell line | Cisplatin | Carboplatin | Oxaliplatin | DMC | Cis-DMC platinum complex | Trans-DMC platinum complex | Trans-S,S(+)-DMC platinum complex | Trans-R,R(-)-DMC platinum complex |
| SK-Hep | 13.45±1.84(3) | 134.23±10.33(3) | 9.09±4.24(2) | 18.96±2.01(5) | 19.41±2.23(1) | 4.27±1.03(3) | 6,15±1.00(4) | 6.63±2.21(3) |
| SK-Hep resistance | 51,78±7.01(2) | 357.65±27.98(2) | | 25.47±1.69(2) | 22.73±1.11(4) | 5.01±0.68(4) | 8.09±0.89(6) | 4.70±0.95(5) |
| Huh-7 | 11.71±1.21(4) | 116.83±9.82(4) | 8.97±4.56(1) | 32.93±3.68(4) | 18.96±1.46(3) | 4.47±0.79(3) | 8.46±1.51(3) | 3.65±1.10(3) |
| Huh-7 resistance | 25.64±3.50(2) | 175.60±53.95(1) | | 26.95±3.03(2) | 17.06±0.77(1) | 5.99±0.52(1) | 16.22±2.60(1) | 26.93±6.14(1) |
| PLC/5 | 6.92±1.46(4) | 80.25±15.78(4) | 6.48±3.19(2) | 25,32±1.98(4) | 17.06±1.18(2) | 4.64±1.01(3) | 6.26±1.87(3) | 3.23±1.13(3) |
| Colo320 | 2.54±0.48(2) | 41.69±5.44(2) | 1.00±0.31(1) | 23.90±1.42(2) | 5.98±0.97(4) | 2.54±0.95(7) | 4.96±1.32(7) | 2.45±0.83(5) |
| L1210 | 0.82±0.14(2) | 12.80±2.14(2) | | 31.23±1.79(7) | | 0.14±0.06(3) | 0.11±0.05(3) | 0.76±0.09(3) |
| L1210 resistance | 13.95±1.49(5) | 126.30±17.23(2) | | 45.53±3.69(5) | | 0.17±0.05(3) | 0.12±0.06(3) | 0.99±0.10(3) |
| HCT-116 | 4.14±0.80(10) | 69.43±14.74(10) | | 23.20±2.06(7) | 12.55±1.67(3) | 0.81±0.29(9) | 2.04±0.41(8) | 0.45±0.27(7) |
| HT-29 | 12.08±2.25(7) | 133.88±15.02(6) | | 32.67±2.57(5) | | 2.09±0.69(9) | 3.07±0.73(8) | 0.92±0.57(6) |

It is suggested by the data of IC₅₀ as shown in Table 5 that:

The antitumor potency in the hepatocellular carcinoma (HCC) cell line of the cis-isomer of the DMC platinum complex is two to three-fold less than that of the trans-S,S-(+)-isomer, trans-R,R-(-)-isomer and trans-racemate of the DMC platinum complex.

SK-Hep shows 4-fold resistance to cisplatin and about 2.7-fold resistance to carboplatin. The trans-S,S-(+)-isomer, trans-R,R-(-)-isomer and trans-racemate of the DMC platinum complex all show effective *in vitro* antitumor activities against SK-Hep.

Huh-7 shows 1.5-fold resistance to both cisplatin and carboplatin. However, Huh-7 shows no cross-resistance to the cis-isomer and trans-racemate of the DMC platinum complex, within experimental errors. The trans-S,S-(+)-isomer, trans-R,R-(-)-isomer and trans-racemate of the DMC platinum complex all show effective *in vitro* antitumor activities against Huh-7 cell.

L1210 shows high resistance to both cisplatin and carboplatin, but shows no cross-resistance to the trans-S,S-(+)-isomer, trans-R,R-(-)-isomer and trans-racemate of the DMC platinum complex.

All stereo-isomers of the DMC platinum complex show effective activities against the colorectal cancer cell line Colo320, in which the trans-R,R-(-)-isomer, trans-racemate and trans-S,S-(+)-isomer of the DMC platinum complex are more effective.

The trans-S,S-(+)-isomer, trans-R,R-(-)-isomer and trans-racemate of the DMC platinum complex all show effective activities against the liver cancer cell line PLC/5, while the potency of the trans-R,R-(-)-isomer of the DMC platinum complex against the liver cancer cell line PLC/5 is the same as that of cisplatin, carboplatin and oxaliplatin.

The potency of the trans-R,R-(-)-isomer against HCT-116 and HT-29 is 10 times more than that of cisplatin.

Since the present invention has been specifically described hereinabove, it will be obvious for those skilled in the art to make various modifications and variations of the invention in view of the above description, and such modifications, variations and equivalents thereof should fall within the scope of the appended claims.

## Claims

1. A stereo-isomer of the demethylcantharidin platinum complex of formula I, 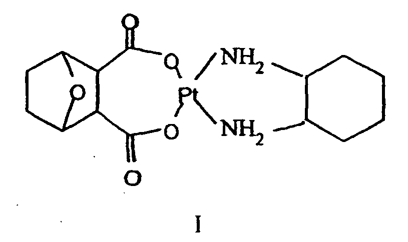 wherein the stereo-isomer is a cis-isomer, a trans-S,S(+)-isomer, a trans-R,R(-)-isomer or a trans-racemate.

2. The stereo-isomer of claim 1, wherein the stereo-isomer is selected from the group consisting of a trans-S,S(+)-isomer, a trans-R,R(-)-isomer and a trans-racemate.

3. Use of a stereo-isomer of the demethylcantharidin platinum complex of formula I, 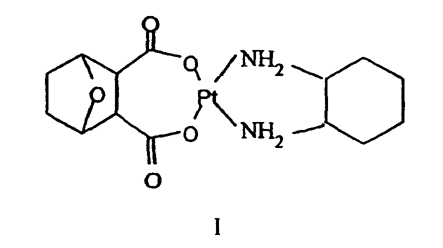 wherein the stereo-isomer is a cis-isomer, a trans-S,S(+)-isomer, a trans-R,R(-)-isomer or a trans-racemate, in manufacturing a medicament for inhibiting the growth of tumorous cells.

4. The use of claim 3, wherein the stereo-isomer is a trans-S,S(+)-isomer, a trans-R,R(-)-isomer or a trans-racemate.

5. The use of claim 3 or 4, wherein the tumorous cells include cisplatin, carboplatin or oxaliplatin-sensitive and -resistant tumorous cells.

6. The use of claim 3 or 4 or 5, wherein the tumorous cells are derived from lung, liver, breast, colon, ovary, cervix, leukemia, lymphoma, or naso-pharyngeal carcinoma.

7. A pharmaceutical composition, comprising a stereo-isomer of the demethylcantharidin platinum complex of formula I, 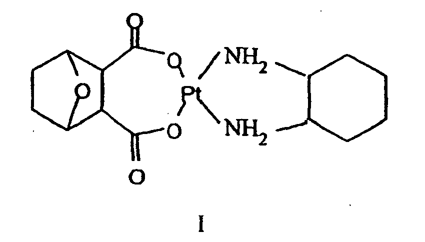 wherein the stereo-isomer is a cis-isomer, a trans-S,S(+)-isomer, a trans-R,R(-)-isomer or a trans-racemate, and a pharmaceutically acceptable carrier.

8. The composition of claim 7, wherein the stereo-isomer is a trans-S,S(+)-isomer, a trans-R,R(-)-isomer or a trans-racemate.

9. The composition of claim 7 or 8, wherein the composition is useful for the treatment of a tumor.

10. The composition of claim 9, wherein the tumor includes cisplatin, carboplatin or oxaliplatin-sensitive and -resistant tumor.

11. The composition of claim 9 or 10, wherein the tumor includes lung, liver, breast, colon, ovary, cervix, leukemia, lymphoma, or naso-pharyngeal carcinoma.

12. The composition of any of claims 7-11, wherein the composition is in a form suitable for a topical administration.

13. The composition of any of claims 7-11, wherein the composition is in a form suitable for an oral administration.

14. The composition of any of claims 7-11, wherein the composition is in a form suitable for an intravenous administration.

15. The composition of any of claims 7-11, wherein the composition is in a form suitable for an intramuscular administration.

16. A method for inhibition of the growth of tumorous cells, comprising contacting the tumorous cells with a stereo-isomer of the demethylcantharidin platinum complex of formula I, 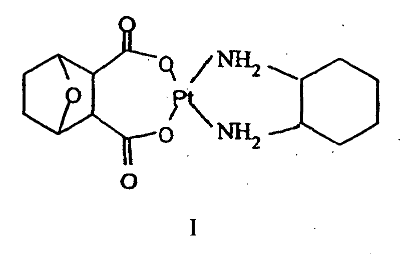 wherein the stereo-isomer is a cis-isomer, a trans-S,S(+)-isomer, a trans-R,R(-)-isomer or a trans-racemate.

17. The method of claim 16, wherein the tumorous cells include cisplatin, carboplatin or oxaliplatin-sensitive and -resistant tumorous cells.

18. The method of claim 16 or 17, wherein the tumorous cells are derived from lung, liver, breast, colon, ovary, cervix, leukemia, lymphoma, or naso-pharyngeal carcinoma.

19. The method of claim 16 or 17 or 18, wherein the stereo-isomer is a trans-S,S(+)-isomer, a trans-R,R(-)-isomer or a trans-racemate.

20. The method of any of claims 16-19, wherein the method is performed *in vitro.*

21. A method for treating a tumor in a subject, comprising administering to the subject a therapeutically effective amount of a stereo-isomer of the demethylcantharidin platinum complex of formula I, 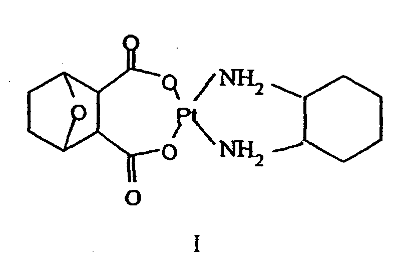 wherein the stereo-isomer is a cis-isomer, a trans-S,S(+)-isomer, a trans-R,R(-)-isomer or a trans-racemate, or a pharmaceutical composition containing the same.

22. The method of claim 21, wherein the tumor includes cisplatin, carboplatin or oxaliplatin-sensitive and -resistant tumor.

23. The method of claim 21 or 22, wherein the tumor includes lung, liver, breast, colon, ovary, cervix, leukemia, lymphoma, or naso-pharyngeal carcinoma.
